# FASCICULE DE BREVET EUROPEEN

(11) **EP 3 517 071 B1**
(45) Date de publication et mention de la délivrance du brevet: **20.04.2022**
(21) Numéro de dépôt: 18154281.2
(22) Date de dépôt: 30.01.2018
(51) Int. Cl.: A61C 7/00, G06T 7/00, G16H 50/00

(54) **SYSTÈME POUR L'ENRICHISSEMENT D'UN MODÈLE NUMÉRIQUE DENTAIRE**
SYSTEM FÜR DIE ANREICHERUNG EINES DIGITALEN GEBISSMODELLS
SYSTEM FOR ENRICHING A DIGITAL DENTAL MODEL

(43) Date de publication de la demande: 31.07.2019
(73) Titulaire: Dental Monitoring, 75008 Paris (FR)
(72) Inventeur: SALAH, Philippe, BAGNOLET 93170 (FR); PELLISSARD, Thomas, 92110 CLICHY (FR)
(74) Mandataire: Nony

(56) Documents cités:
- WO-A1-2017/182654
- FR-A- 1 460 310
- US-A1- 2008 318 179
- US-A1- 2015 320 320

## Description

### Domaine technique

La présente invention concerne un système pour 1"enrichissement d'un modèle numérique d'une arcade dentaire d'un patient.

### Etat de la technique

Il est souhaitable que chacun fasse régulièrement contrôler sa dentition, notamment afin de vérifier que la position de ses dents n'évolue pas défavorablement. Lors d'un traitement orthodontique, cette évolution défavorable peut notamment conduire à modifier le traitement. Après un traitement orthodontique, cette évolution défavorable, appelée « récidive », peut conduire à une reprise d'un traitement. Enfin, de manière plus générale et indépendamment de tout traitement, chacun peut souhaiter suivre les déplacements éventuels de ses dents.

Classiquement, les contrôles sont effectués par un orthodontiste ou un dentiste qui, seuls, disposent d'un appareillage adapté. Ces contrôles sont donc coûteux. En outre, les visites sont contraignantes.

US2008318179 divulgue une méthode de correction d'un modèle dentaire.

US 2009/0291417 décrit un procédé permettant de créer, puis de modifier des modèles tridimensionnels, notamment pour la fabrication d'appareils orthodontiques.

WO 2016 066651 décrit un procédé de contrôle du positionnement et/ou de la forme et/ou de l'aspect de dents d'un patient. Ce procédé comporte une étape de création d'un modèle de référence initial des dents, de préférence avec un scanner 3D, puis, à un instant ultérieur, la création d'un modèle de référence actualisé, par déformation du modèle de référence initial. Cette déformation est effectuée de manière que le modèle de référence actualisé permette des vues les plus concordantes possible avec les images des dents acquises à l'instant ultérieur, en particulier des photos ou des images d'une vidéo prises par le patient lui-même.

La déformation du modèle de référence initial correspond donc à une évolution de la position et/ou de la forme des dents entre la création du modèle de l'instant initial de la création du modèle de référence initial et l'instant ultérieur. Cette évolution peut avoir conduit à l'apparition de régions de dents qui étaient initialement enfouies dans la gencive, ou cachées par d'autres dents, ou à l'apparition de régions de la gencive qui n'étaient pas visibles lors de la création du modèle de référence initial.

Le modèle de référence actualisé présente donc des « zones blanches » dans les régions non observables à l'instant initial. Il est donc incomplet et ne permet donc pas, en particulier, de détecter ultérieurement une déformation d'une dent ou de la gencive dans ces régions. Ce problème est particulièrement critique lorsque le modèle de référence actualisé est exploité pour la fabrication d'un appareil orthodontique, par exemple dans le cadre d'une reprise d'un traitement défectueux, ou pour la réalisation d'un appareil orthodontique de contention post-traitement.

Il existe un besoin pour un procédé permettant de limiter les zones blanches d'un modèle, en particulier dans les modèles de référence décrits dans WO 2016 066651.

Un but de l'invention est de répondre, au moins partiellement, à ce besoin.

### Résumé de l'invention

L'invention propose un système selon la revendication 1.

On appelle ci-dessous « procédé d'enrichissement » un procédé d'enrichissement d'un modèle de référence à enrichir représentant une arcade dentaire d'un patient, ledit procédé comportant les étapes suivantes :
A) acquisition, dans des premières conditions d'acquisition réelles, d'une première image actualisée de ladite arcade faisant apparaître une région de ladite arcade ;
B) exploration du modèle de référence à enrichir de manière à déterminer une première vue du modèle de référence à enrichir, suivant une première direction d'observation, ou « première image de référence », ladite première image de référence présentant une concordance maximale avec la première image actualisée ;
C) détermination, par comparaison de la première image de référence et de la première image actualisée, d'un premier point orphelin représenté sur la première image actualisée et non représenté sur la première image de référence lorsque la première image actualisée est dans une première position de registre dans laquelle elle est superposée, dans l'espace du modèle de référence à enrichir, avec la première image de référence ;
D) ajout, dans le modèle de référence à enrichir, d'un point, dit « point parent », sur une première droite parallèle à la première direction d'observation et passant par le premier point orphelin dans la première position de registre ;
E) optionnellement, comparaison du modèle de référence à enrichir avec le modèle de référence enrichi.

Le produit programme d'ordinateur d'un système selon l'invention comprend donc des instructions de code de programme pour la mise en œuvre, lorsque le programme est exécuté par ordinateur, des étapes B) à D), et optionnellement E).

De préférence, la position du point parent sur la première droite est
(a) la position la plus proche d'une deuxième droite déterminée suivant les étapes suivantes :
   - acquisition, dans des deuxièmes conditions d'acquisition réelles, d'une deuxième image actualisée de ladite arcade faisant apparaître ladite région de ladite arcade ;
   - exploration du modèle de référence à enrichir de manière à déterminer une deuxième vue du modèle de référence à enrichir, suivant une deuxième direction d'observation, ou «deuxième image de référence », ladite deuxième image de référence présentant une concordance maximale avec la deuxième image actualisée ;
   - détermination d'un deuxième point orphelin présentant les mêmes coordonnées que le premier point orphelin dans un référentiel commun aux première et deuxième images actualisées, la deuxième droite étant la droite parallèle à la deuxième direction d'observation et passant par le deuxième point orphelin dans une deuxième position de registre dans laquelle la deuxième image actualisée est superposée, dans l'espace du modèle de référence à enrichir, avec la deuxième image de référence ; ou
(b) déterminée en fonction d'une distance avec une surface approximative et/ou une ligne approximative définie, dans l'espace du modèle de référence à enrichir.

Comme on le verra plus en détail dans la suite de la description, un système selon l'invention permet un enrichissement rapide et fiable du modèle de référence.

Un système selon l'invention peut encore notamment comporter une ou plusieurs des caractéristiques optionnelles suivantes :
- dans le mode de réalisation (a), le référentiel commun est déterminé par au moins trois premiers points remarquables sur la première image actualisée et trois deuxièmes points remarquables sur la deuxième image actualisée, chaque couple d'un premier point remarquable et d'un deuxième point remarquable représentant un même point remarquable de l'arcade ;
- un point remarquable de l'arcade est un point présentant une particularité, de préférence facilement identifiable par un opérateur, de préférence un point où un contour de l'arcade évolue de manière atypique, par exemple en formant un angle aigüe, ou se ramifie ;
- dans le mode de réalisation (b), le point parent est positionné de manière à minimiser ladite distance avec la surface approximative et/ou la ligne approximative ;
- la surface approximative et/ou la ligne approximative sont déterminées par des méthodes statistiques ou au moyen d'un réseau de neurones ;
- on recherche la première et/ou deuxième image de référence au moyen d'une méthode métaheuristique ;
- pour déterminer au moins une desdites première et deuxième images de référence, on recherche des conditions d'acquisition virtuelles dans lesquelles l'observation du modèle de référence à enrichir fournit une image présentant une concordance maximale avec ladite au moins une desdites première et deuxième images actualisées, respectivement ;
- on détermine ladite image de référence suivant les étapes suivantes :
   a. traitement de l'image actualisée pour réaliser au moins une carte actualisée représentant, au moins partiellement, une information discriminante ;
   b. détermination de conditions d'acquisition virtuelles à tester ;
   c. réalisation d'une image de référence du modèle de référence à enrichir dans lesdites conditions d'acquisition virtuelles à tester, ou « image de référence à tester »;
   d. traitement de l'image de référence à tester pour réaliser au moins une carte de référence représentant ladite information discriminante ;
   e. comparaison des cartes actualisée et de référence de manière à déterminer une valeur pour une première fonction d'évaluation, ladite valeur pour la première fonction d'évaluation dépendant des différences entre lesdites cartes actualisée et de référence ;
   f. en fonction de la valeur de la première fonction d'évaluation, modification des conditions d'acquisition virtuelles à tester, puis reprise à l'étape c. ou
      définition de ladite image de référence comme étant l'image de référence à tester ;
- l'information discriminante est choisie dans le groupe constitué par une information de contour, une information de couleur, une information de densité, une information de distance, une information de brillance, une information de saturation, une information sur les reflets et des combinaisons de ces informations ;
- la détermination des conditions d'acquisition virtuelles à tester est effectuée au moyen d'une méthode métaheuristique ;
- le modèle de référence à enrichir est généré par un scan de ladite arcade du patient ;
- les étapes B) à D) sont exécutées, en boucle, pour plus de 5, de préférence plus de 10, de préférence plus de 100 couples de première et deuxième images actualisées ;
- les étapes B) à D) sont exécutées, en boucle, pour chaque point de la première image actualisée non représenté dans le modèle de référence à enrichir tel que défini avant la première occurrence de l'étape B) ;
- à l'étape E), on détermine, à partir de la comparaison du modèle de référence à enrichir avec le modèle de référence enrichi, une évolution de la forme et/ou de la position d'une dent de l'arcade dentaire, et/ou une perte de matière résultant d'une abrasion de ladite dent ;
- les première et deuxième images actualisées sont acquises plus de 2, plus de 5 ou plus de 10 semaines avant ou après la génération du modèle de référence à enrichir.

Un système selon l'invention peut être en particulier mis en œuvre pour enrichir un modèle de référence actualisé obtenu par déformation d'un modèle de référence initial, de préférence suivant l'enseignement de WO 2016 066651.

Il procède de préférence suivant les étapes suivantes :
1) à un instant initial, réalisation d'un modèle de référence d'une arcade d'un patient, ou « modèle de référence initial » ;
2) à un instant actualisé, acquisition desdites première et deuxième images actualisées ;
3) déformation du modèle de référence initial de manière à obtenir un modèle de référence actualisé à partir duquel des première et deuxième images de référence en concordance maximale avec les première et deuxième images actualisées, respectivement, sont observables ;
4) enrichissement du modèle de référence actualisé à partir desdites première et deuxième images de référence, suivant les étapes ii) à iv).

De préférence, à l'étape 3), on détermine la déformation du modèle de référence initial au moyen d'une méthode métaheuristique.

Un système selon l'invention peut notamment être mis en œuvre dans le cadre d'un procédé de contrôle du positionnement et/ou de la forme et/ou de l'aspect de dents d'un patient tel que celui décrit dans WO 2016 066651.

### Définitions

Un « patient » est une personne pour laquelle un dit procédé d'enrichissement est mis en œuvre, indépendamment du fait que cette personne suive un traitement orthodontique ou non.

Par « orthodontiste », on entend toute personne qualifiée pour prodiguer des soins dentaires, ce qui inclut également un dentiste.

Par « pièce orthodontique », on entend tout ou partie d'un appareil orthodontique.

Par « modèle », on entend un modèle tridimensionnel numérique. On distingue le « modèle de référence à enrichir », avant mise en œuvre du procédé de l'invention, et le « modèle de référence enrichi », issu de la mise en œuvre du procédé de l'invention. Un « modèle de référence actualisé » est un modèle obtenu par déformation optimale d'un « modèle de référence initial » à partir d'images, comme décrit dans WO 2016 066651. Un modèle de référence actualisé est un exemple particulier d'un modèle de référence à enrichir.

Par "image", on entend une image en deux dimensions, comme une photographie ou une image extraite d'un film. Une image est formée de pixels.

Une « image de référence » est une vue d'un modèle « de référence ». Selon l'invention, on cherche une image de référence qui présente une concordance maximale avec une image actualisée. A cet effet, on se place dans l'espace du modèle de référence et on explore cet espace en observant ce modèle, jusqu'à pouvoir sensiblement observer l'image actualisée. Autrement dit, si on superpose le mieux possible l'image actualisée avec la vue du modèle ainsi obtenue (image de référence), on obtient, en dehors des zones blanches, une superposition sensiblement parfaite, dite « en registre ». L'image actualisée en registre avec ladite image de référence, dans l'espace du modèle, est dite en « position de registre ». L'observation des images actualisée et de référence en position de registre permet, par transparence, d'identifier les points de l'image actualisée qui ne sont pas représentés sur l'image de référence, c'est-à-dire les points orphelins qui ne sont pas représentés dans le modèle de référence.

Par « image d'une arcade », ou « modèle d'une arcade », on entend une représentation de tout ou partie de ladite arcade.

Les « conditions d'acquisition » d'une image précisent la position et l'orientation dans l'espace d'un appareil d'acquisition de cette image relativement aux dents du patient (conditions d'acquisition réelles) ou à un modèle de dents du patient (conditions d'acquisition virtuelles), et de préférence la calibration de cet appareil d'acquisition. Des conditions d'acquisition sont dites "virtuelles" lorsqu'elles correspondent à une simulation dans laquelle l'appareil d'acquisition serait dans lesdites conditions d'acquisition (positionnement et de préférence calibration théoriques de l'appareil d'acquisition) par rapport à un modèle.

Dans des conditions d'acquisition virtuelles d'une image de référence, l'appareil d'acquisition peut être également qualifié de « virtuel ». L'image de référence est en effet acquise par un appareil d'acquisition fictif, ayant les caractéristiques de l'appareil d'acquisition « réel » ayant servi à l'acquisition des images réelles, et en particulier des images actualisées.

La « calibration » d'un appareil d'acquisition est constituée par l'ensemble des valeurs des paramètres de calibration. Un "paramètre de calibration" est un paramètre intrinsèque à l'appareil d'acquisition (à la différence de sa position et de son orientation) dont la valeur influence l'image acquise. De préférence, les paramètres de calibration sont choisis dans le groupe formé par l'ouverture de diaphragme, le temps d'exposition, la distance focale et la sensibilité.

Une "information discriminante" est une information caractéristique qui peut être extraite d'une image (*"image feature"*), classiquement par un traitement informatique de cette image.

Une information discriminante peut présenter un nombre variable de valeurs. Par exemple, une information de contour peut être égale à 1 ou 0 selon qu'un pixel appartient ou non à un contour. Une information de brillance peut prendre un grand nombre de valeurs. Le traitement de l'image permet d'extraire et de quantifier l'information discriminante.

L'information discriminante peut être représentée sous la forme d'une « carte ». Une carte est ainsi le résultat d'un traitement d'une image afin de faire apparaître l'information discriminante, par exemple le contour des dents et des gencives.

Un point d'une image (ou d'une carte), par exemple un point d'un contour de dent, est issu de la projection d'un point de l'arcade dentaire du patient, suivant la direction de prise de vue lors de l'acquisition de l'image (la direction de prise de vue étant définie par l'orientation de l'objectif de l'appareil d'acquisition d'images lors de cette acquisition). Le point du modèle de l'arcade dentaire représentant ce point de l'arcade dentaire est appelé « point parent » du point de l'image (ou d'une carte).

Autrement dit, le point de l'image est la représentation du point parent dans les conditions d'acquisition de l'image. En particulier le point de la carte actualisée est la représentation du point parent dans les conditions d'acquisition de l'image actualisée.

Lorsqu'un point d'une image, par exemple un point de contour, n'a pas de point parent dans le modèle, il est dit « orphelin ». Le modèle présente donc une zone blanche à l'emplacement du point parent manquant.

Un point parent est à un emplacement « compatible » avec un point d'une image lorsqu'il est représenté, sur cette image, par ce point.

On appelle «concordance» (*«match»* ou *«fit» en* anglais) entre deux objets une mesure de la différence entre ces deux objets. Une concordance est maximale («*best fit»*) lorsqu'elle résulte d'une optimisation de manière à minimiser ladite différence.

Un objet modifié pour obtenir une concordance maximale peut être qualifié d'objet « optimal ».

Deux images ou « vues » qui présentent une concordance maximale représentent sensiblement au moins une même dent, de la même façon. Autrement dit, les représentations de la dent sur ces deux images sont sensiblement superposables.

La recherche d'une image de référence présentant une concordance maximale avec une image actualisée s'effectue en recherchant les conditions d'acquisition virtuelles de l'image de référence présentant une concordance maximale avec les conditions d'acquisition réelles de l'image actualisée.

Par extension, un modèle présente une concordance maximale avec une image lorsque ce modèle a été choisi parmi plusieurs modèles parce qu'il permet une vue présentant une concordance maximale avec ladite image et/ou lorsque cette image a été choisie parmi plusieurs images parce qu'elle présente une concordance maximale avec une vue dudit modèle.

En particulier, une image actualisée est en concordance maximale avec un modèle de référence lorsqu'une vue de ce modèle de référence fournit une image de référence en concordance maximale avec l'image actualisée.

La comparaison entre deux images résulte de préférence de la comparaison de deux cartes correspondantes. On appelle classiquement « « distance » une mesure de la différence entre deux cartes ou entre deux images.

Les méthodes « métaheuristiques » sont des méthodes d'optimisation connues. Elles sont de préférence choisies dans le groupe formé par
- les algorithmes évolutionnistes, de préférence choisie parmi: les stratégies d'évolution, les algorithmes génétiques, les algorithmes à évolution différentielle, les algorithmes à estimation de distribution, les systèmes immunitaires artificiels, la recomposition de chemin Shuffled Complex Evolution, le recuit simulé, les algorithmes de colonies de fourmis, les algorithmes d'optimisation par essaims particulaires, la recherche avec tabous, et la méthode GRASP ;
- l'algorithme du kangourou,
- la méthode de Fletcher et Powell,
- la méthode du bruitage,
- la tunnelisation stochastique,
- l'escalade de collines à recommencements aléatoires,
- la méthode de l'entropie croisée, et
- les méthodes hybrides entre les méthodes métaheuristiques citées ci-dessus.

Il faut interpréter "comprenant " ou "comportant " ou "présentant " de manière non restrictive, sauf indication contraire.

### Brève description des figures

D'autres caractéristiques et avantages de l'invention apparaîtront encore à la lecture de la description détaillée qui va suivre et à l'examen du dessin annexé dans lequel :
- la figure 1 représente, schématiquement, les différentes étapes d'un enrichissement d'un modèle ;

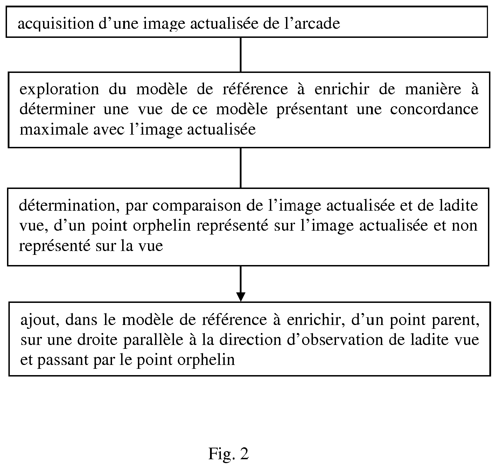

- la figure 2 représente un logigramme illustrant la mise en œuvre d'un dit procédé d'enrichissement ;
- la figure 3 représente un logigramme illustrant une recherche d'un modèle de référence actualisé à partir d'un modèle de référence initial ;
- la figure 4 représente un exemple d'une image de référence d'un modèle de référence initial,
- la figure 5 (5a-5d) illustre un traitement pour déterminer les modèles de dent dans un modèle de référence initial, comme décrit dans WO 2016 066651,
- la figure 6 (6a-6d) illustre l'acquisition d'une image actualisée au moyen d'un écarteur, une opération de découpage de cette image, et le traitement d'une image actualisée permettant de déterminer le contour des dents, comme décrit dans WO 2016 066651,
- la figure 7 illustre schématiquement la position relative de marques de repérage 12 d'un écarteur 10 sur des images actualisées 141 et 142, selon les directions d'observation représentées en traits interrompus.

### Description détaillée

Un dit procédé d'enrichissement est destiné à enrichir un modèle. Il peut être en particulier mis en œuvre dans le cadre d'un procédé comportant des étapes 1) à 4).

**L'étape 1)** est destinée à la réalisation d'un modèle de référence initial modélisant une arcade du patient. Elle comprend de préférence une ou plusieurs des caractéristiques de l'étape a) de WO 2016 066651.

Le modèle de référence initial est de préférence créé avec un scanner 3D. Un tel modèle, dit « 3D », peut être observé selon un angle quelconque. Une observation du modèle, selon un angle et à une distance déterminés, est appelée une « vue » ou « image de référence ».

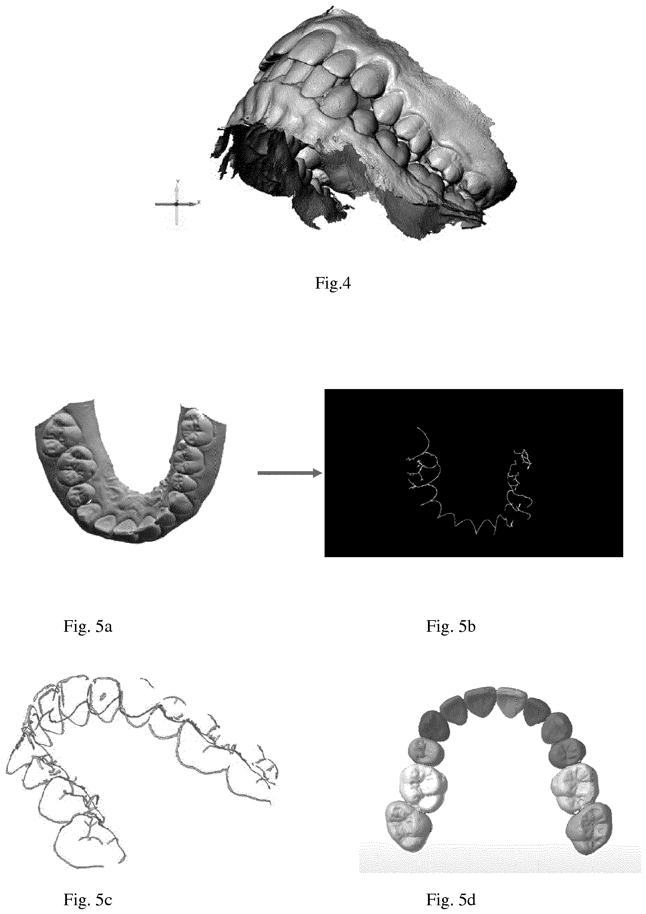

La figure 4 est un exemple d'image de référence.

Le modèle de référence initial peut être préparé à partir de mesures effectuées sur les dents du patient ou sur un moulage de ses dents, par exemple un moulage en plâtre.

De préférence, pour chaque dent, on définit, à partir du modèle de référence initial, un modèle de ladite dent, ou « modèle de dent » (figure 5). Cette opération, connue en elle-même, est également appelée « segmentation du modèle de référence initial ».

Dans le modèle de référence initial, un modèle de dent est délimité par un bord gingival qui peut être décomposé en un bord gingival intérieur (du côté de l'intérieur de la bouche par rapport à la dent), un bord gingival extérieur (orienté vers l'extérieur de la bouche par rapport à la dent) et deux bords gingivaux latéraux.

Dans le modèle de référence initial, seule la surface exposée à l'extérieur peut être scannée. Par exemple, les surfaces de deux dents adjacentes qui se font face peuvent être inaccessibles au scanner, et ne pas être représentées. Il en est de même des parties de dents qui sont recouvertes par la gencive. Le modèle de référence initial ne représente donc pas l'intégralité des dents et de la gencive. Les « trous » dans le modèle sont appelées « zones blanches ». Si un scan complet de l'arcade dentaire a été effectué, le modèle de référence initial ne fait cependant pas apparaitre les zones blanches.

L'instant initial auquel le modèle de référence initial est généré peut être en particulier un instant précédant un traitement orthodontique actif, par exemple moins de 6 mois, moins de 3 mois, ou moins de 1 mois avant le début du traitement. Les étapes 1) à 3) sont alors mises en œuvre pour suivre l'évolution du traitement entre l'instant initial et l'instant actualisé de l'étape 2).

L'instant initial peut être alternativement un instant en fin de traitement orthodontique actif, par exemple moins de 6 mois, moins de 3 mois, ou moins de 1 mois après la fin du traitement. Les étapes 1) à 3) sont alors mises en œuvre pour surveiller l'apparition d'une récidive éventuelle.

**L'étape 2**) est destinée à l'acquisition, à un instant actualisé, d'images actualisées destinées à guider la modification du modèle de référence initial pour définir le modèle de référence actualisé, à l'étape 3). Pour l'enrichissement de ce modèle, au moins des première et deuxième images actualisées de l'arcade doivent être acquises, dans des premières et deuxièmes conditions d'acquisition réelles faisant apparaître, sous des angles d'observation différents, une même région de ladite arcade.

L'étape 2) comprend de préférence une ou plusieurs des caractéristiques de l'étape b) de WO 2016 066651.

L'acquisition des images actualisées est réalisée au moyen d'un appareil d'acquisition d'images, de préférence d'un téléphone mobile.

L'intervalle de temps entre les étapes 1) et 2) peut être par exemple supérieur à 1 semaine, à 2 semaines, à 1 mois, à 2 mois ou à 6 mois.

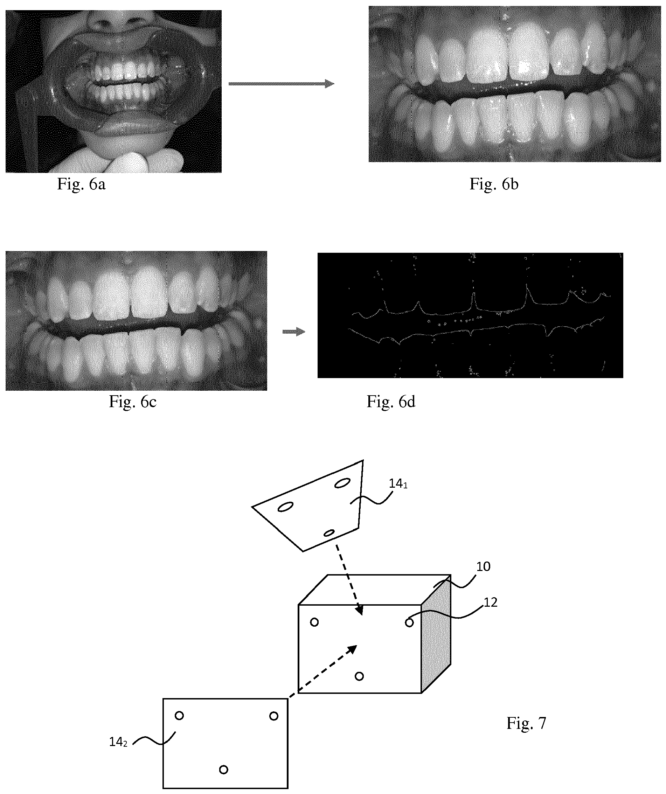

De préférence, on utilise un écarteur dentaire 10 lors de l'étape 2), comme représenté sur la figure 6a. L'écarteur comporte classiquement un support muni d'un rebord s'étendant autour d'une ouverture et agencé de manière que les lèvres du patient puissent y reposer en laissant les dents du patient apparaître à travers ladite ouverture.

**L'étape 3**) est destinée à la génération d'un modèle de référence actualisé présentant une concordance maximale avec les images actualisées.

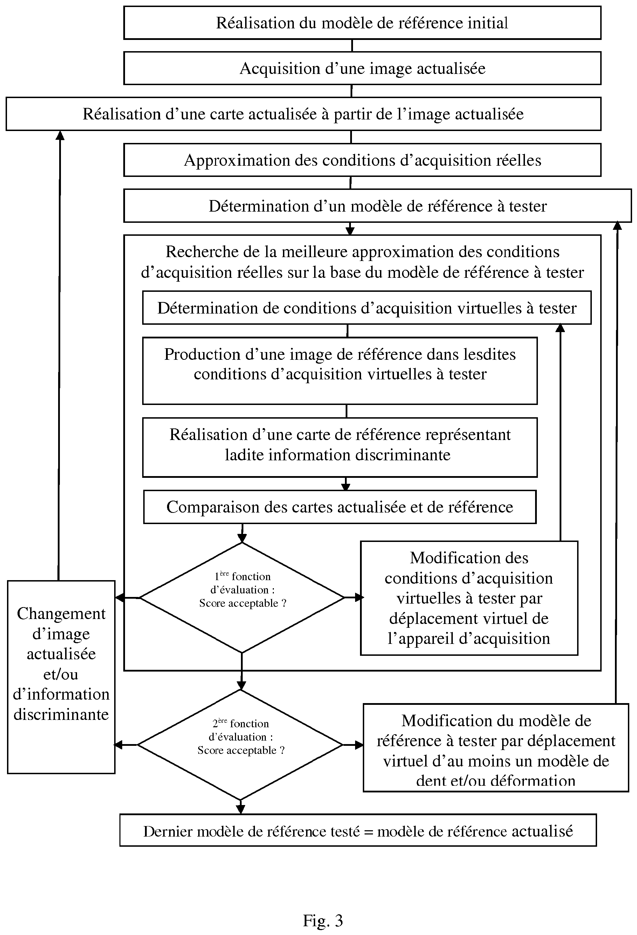

Elle comprend de préférence une ou plusieurs des caractéristiques des étapes c), d) et e) de WO 2016 066651, comme illustré sur la figure 3.

A l'étape 3), chaque image actualisée est analysée de manière à réaliser, pour chaque image actualisée, une carte actualisée relative à au moins une information discriminante.

Une carte actualisée représente une information discriminante dans le référentiel de l'image actualisée. Par exemple, la figure 5b est une carte actualisée relative au contour des dents obtenue à partir de l'image actualisée de la figure 5a.

L'information discriminante est de préférence choisie dans le groupe constitué par une information de contour, une information de couleur, une information de densité, une information de distance, une information de brillance, une information de saturation, une information sur les reflets et des combinaisons de ces informations.

L'homme de l'art sait comment traiter une image actualisée pour faire apparaître l'information discriminante.

Ensuite, pour chaque image actualisée, on détermine de préférence, de manière grossière, des conditions d'acquisition virtuelles approximant les conditions d'acquisition réelles lors de l'étape 1). Autrement dit, on estime la position de l'appareil d'acquisition d'images par rapport aux dents au moment où il a pris l'image actualisée (position de l'appareil d'acquisition dans l'espace et orientation de cet appareil). Cette évaluation grossière permet avantageusement de limiter le nombre de tests sur des conditions d'acquisition virtuelles lors des opérations suivantes, et permet donc d'accélérer considérablement ces opérations.

Pour effectuer cette évaluation grossière, on utilise de préférence une ou plusieurs règles heuristiques. Par exemple, de préférence, on exclut des conditions d'acquisition virtuelles susceptibles d'être testées lors des opérations suivantes, les conditions qui correspondent à une position de l'appareil d'acquisition d'images derrière les dents ou à une distance des dents supérieure à 1 m.

Dans un mode de réalisation préféré, comme illustré sur la figure 7, on utilise des marques de repérage représentées sur l'image actualisée, et en particulier des marques de repérage 12 de l'écarteur, pour déterminer une région de l'espace sensiblement conique délimitant des conditions d'acquisition virtuelles susceptibles d'être testées lors des opérations suivantes, ou "cône de test".

Précisément, on dispose de préférence au moins trois marques de repérage 12 non alignées sur l'écarteur 10, et on mesure précisément leurs positions relatives sur l'écarteur.

Les marques de repérage sont ensuite repérées sur l'image actualisée, comme décrit précédemment. De simples calculs trigonométriques permettent de déterminer approximativement la direction selon laquelle l'image actualisée a été prise.

Ensuite, on recherche finement, pour chaque image actualisée, un modèle de référence actualisé correspondant au positionnement et/ou à la forme des dents lors de l'acquisition de l'image actualisée, la recherche étant de préférence effectuée au moyen d'une méthode métaheuristique, de préférence évolutionniste, de préférence par recuit simulé.

Cette recherche est de préférence réalisée à partir des conditions d'acquisition virtuelles évaluées grossièrement.

L'objectif est de modifier le modèle de référence initial jusqu'à obtenir un modèle de référence actualisé qui présente une concordance maximale avec l'image actualisée. Idéalement, le modèle de référence actualisé est donc un modèle de référence à partir duquel l'image actualisée aurait pu être prise si ce modèle de l'arcade avait été l'arcade elle-même.

On teste donc une succession de modèles de référence « à tester », le choix d'un modèle de référence à tester étant de préférence dépendant du niveau de correspondance des modèles de référence «à tester» précédemment testés avec l'image actualisée. Ce choix est de préférence effectué en suivant un procédé d'optimisation connu, en particulier choisi parmi les procédés d'optimisation métaheuristiques, de préférence évolutionnistes, en particulier dans les procédés de recuit simulé.

De préférence, la recherche comporte
- une première opération d'optimisation permettant de rechercher des conditions d'acquisition virtuelles correspondant au mieux aux conditions d'acquisition réelles dans un modèle de référence à tester déterminé à partir du modèle de référence initial, et
- une deuxième opération d'optimisation permettant de rechercher, en testant une pluralité de dits modèles de référence à tester, le modèle de référence correspondant au mieux au positionnement des dents du patient lors de l'acquisition de l'image actualisée à l'étape 1).

De préférence, une première opération d'optimisation est effectuée pour chaque test d'un modèle de référence à tester lors de la deuxième opération d'optimisation.

De préférence, la première opération d'optimisation et/ou la deuxième opération d'optimisation, de préférence la première opération d'optimisation et la deuxième opération d'optimisation mettent en œuvre une méthode métaheuristique, de préférence évolutionniste, de préférence un recuit simulé.

De préférence, la recherche, pour chaque image actualisée, d'un modèle de référence actualisé, correspondant à l'étape e) de WO 2016 066651, comporte les étapes suivantes :
e1) définition d'un modèle de référence à tester comme étant le modèle de référence initial puis,
e2) suivant les étapes suivantes, test de conditions d'acquisition virtuelles avec le modèle de référence à tester afin d'approximer finement lesdites conditions d'acquisition réelles ;
   e21) détermination de conditions d'acquisition virtuelles à tester;
   e22) réalisation d'une image de référence du modèle de référence à tester dans lesdites conditions d'acquisition virtuelles à tester ;
   e23) traitement de l'image de référence pour réaliser au moins une carte de référence représentant, au moins partiellement, l'information discriminante ;
   e24) comparaison des cartes actualisée et de référence de manière à déterminer une valeur pour une première fonction d'évaluation, ladite valeur pour la première fonction d'évaluation dépendant des différences entre lesdites cartes actualisée et de référence et correspondant à une décision de poursuivre ou de stopper la recherche de conditions d'acquisition virtuelles approximant lesdites conditions d'acquisition réelles avec plus d'exactitude que lesdites conditions d'acquisition virtuelles à tester déterminées à la dernière occurrence de l'étape e21) ;
   e25) si ladite valeur pour la première fonction d'évaluation correspond à une décision de poursuivre ladite recherche, modification des conditions d'acquisition virtuelles à tester, puis reprise à l'étape e22) ;
e3) détermination d'une valeur pour une deuxième fonction d'évaluation, ladite valeur pour la deuxième fonction d'évaluation dépendant des différences entre les cartes actualisée et de référence dans les conditions d'acquisition virtuelles approximant au mieux lesdites conditions d'acquisition réelles et résultant de la dernière occurrence de l'étape e2), ladite valeur pour la deuxième fonction d'évaluation correspondant à une décision de poursuivre ou de stopper la recherche d'un modèle de référence approximant le positionnement des dents lors de l'acquisition de l'image actualisée avec plus d'exactitude que ledit modèle de référence à tester utilisé à la dernière occurrence de l'étape e2), et si ladite valeur pour la deuxième fonction d'évaluation correspond à une décision de poursuivre ladite recherche, modification du modèle de référence à tester par déplacement d'un ou plusieurs modèles de dents, puis reprise à l'étape e2).

**A l'étape e1**)**,** on détermine que le modèle de référence à tester est le modèle de référence initial lors de la première exécution de l'étape e2).

**A l'étape e2**), on commence par déterminer des conditions d'acquisition virtuelles à tester, c'est-à-dire une position et une orientation virtuelles susceptibles de correspondre à la position et l'orientation réelles de l'appareil d'acquisition lors de la capture de l'image actualisée, mais aussi, de préférence, une calibration virtuelle susceptible de correspondre à la calibration réelle de l'appareil d'acquisition lors de la capture de l'image actualisée.

On configure ensuite virtuellement l'appareil d'acquisition d'images dans les conditions d'acquisition virtuelles à tester afin d'acquérir une image de référence du modèle de référence à tester dans ces conditions d'acquisition virtuelles à tester. L'image de référence correspond donc à l'image qu'aurait prise l'appareil d'acquisition d'images s'il avait été placé, par rapport au modèle de référence à tester, et optionnellement calibré, dans les conditions d'acquisition virtuelles à tester (étape e22)).

Si l'image actualisée a été prise alors que la position des dents était exactement celle dans le modèle de référence à tester, et si les conditions d'acquisition virtuelles sont exactement les conditions d'acquisition réelles, l'image de référence est donc exactement superposable à l'image actualisée. Les différences entre l'image actualisée et l'image de référence résultent d'erreurs dans l'évaluation des conditions d'acquisition virtuelles (si elles ne correspondent pas exactement aux conditions d'acquisition réelles) et de différences de positionnement des dents entre l'étape 2) et le modèle de référence à tester.

Pour comparer les images actualisée et de référence, on compare l'information discriminante de ces deux images. Plus précisément, on réalise, à partir de l'image de référence, une carte de référence représentant l'information discriminante (étape e23)).

Les cartes actualisée et de référence, portant toutes les deux sur la même information discriminante, sont ensuite comparées et on évalue la différence entre ces deux cartes au moyen d'un score. Par exemple, si l'information discriminante est le contour des dents, on peut comparer la distance moyenne entre les points du contour des dents qui apparait sur l'image de référence et les points du contour correspondant qui apparaît sur l'image actualisée, le score étant d'autant plus élevé que cette distance est faible.

De préférence, les conditions d'acquisition virtuelles comprennent les paramètres de calibration de l'appareil d'acquisition. Le score est d'autant plus élevé que les valeurs des paramètres de calibration testées sont proches des valeurs des paramètres de calibration de l'appareil d'acquisition utilisé lors de l'acquisition de l'image actualisée. Par exemple, si l'ouverture de diaphragme testée est éloignée de celle de l'appareil d'acquisition utilisé lors de l'acquisition de l'image actualisée, l'image de référence présente des régions floues et des régions nettes qui ne correspondent pas aux régions floues et aux régions nettes de l'image actualisée. Si l'information discriminante est le contour des dents, les cartes actualisée et de référence ne représenteront donc pas les mêmes contours et le score sera faible.

Le score peut être par exemple un coefficient de corrélation.

Le score est ensuite évalué au moyen d'une première fonction d'évaluation. La première fonction d'évaluation permet de décider si le cyclage sur l'étape e2) doit être poursuivi ou stoppé. La première fonction d'évaluation peut par exemple être égale à 0 si le cyclage doit être stoppé ou être égale à 1 si le cyclage doit se poursuivre.

La valeur de la première fonction d'évaluation peut dépendre du score atteint. Par exemple, il peut être décidé de poursuivre le cyclage sur l'étape e2) si le score ne dépasse pas un premier seuil. Par exemple, si une correspondance exacte entre les images actualisée et de référence conduit à un score de 100%, le premier seuil peut être, par exemple, de 95%. Bien entendu, plus le premier seuil sera élevé, meilleure sera la précision de l'évaluation des conditions d'acquisition virtuelles si le score parvient à dépasser ce premier seuil.

La valeur de la première fonction d'évaluation peut également dépendre de scores obtenus avec des conditions d'acquisition virtuelles testées précédemment.

La valeur de la première fonction d'évaluation peut également dépendre de paramètres aléatoires et/ou du nombre de cycles de l'étape e2) déjà effectués.

En particulier, il est possible qu'en dépit de la répétition des cycles, on ne parvienne pas à trouver des conditions d'acquisition virtuelles qui soient suffisamment proches des conditions d'acquisition réelles pour que le score atteigne ledit premier seuil. La première fonction d'évaluation peut alors conduire à la décision de quitter le cyclage bien que le meilleur score obtenu n'ait pas atteint ledit premier seuil. Cette décision peut résulter, par exemple, d'un nombre de cycles supérieur à un nombre maximal prédéterminé.

Un paramètre aléatoire dans la première fonction d'évaluation peut également autoriser la poursuite de tests de nouvelles conditions d'acquisition virtuelles, bien que le score apparaisse satisfaisant.

Les fonctions d'évaluation classiquement utilisées dans les procédés d'optimisation métaheuristiques, de préférence évolutionnistes, en particulier dans les procédés de recuit simulé, peuvent être utilisées pour la première fonction d'évaluation.

Si la valeur de la première fonction d'évaluation indique qu'il est décidé de poursuivre le cyclage sur l'étape e2), on modifie les conditions d'acquisition virtuelles à tester (étape e25)) et on recommence un cycle (étape e2)) consistant à réaliser une image de référence et une carte de référence, puis à comparer cette carte de référence avec la carte actualisée pour déterminer un score.

La modification des conditions d'acquisition virtuelles correspond à un déplacement virtuel dans l'espace et/ou à une modification de l'orientation et/ou, de préférence, à une modification de la calibration de l'appareil d'acquisition. Cette modification peut être aléatoire, à condition cependant que les nouvelles conditions d'acquisition virtuelles à tester appartiennent toujours à l'ensemble déterminé lors de l'évaluation grossière. La modification est de préférence guidée par des règles heuristiques, par exemple en favorisant les modifications qui, d'après une analyse des précédents scores obtenus, apparaissent les plus favorables pour augmenter le score.

Le cyclage sur e2) est poursuivi jusqu'à ce que la valeur de la première fonction d'évaluation indique qu'il est décidé de sortir de ce cyclage et de poursuivre à l'étape e3), par exemple si le score atteint ou dépasse ledit premier seuil.

L'optimisation des conditions d'acquisition virtuelles à l'étape e2) est de préférence effectuée en utilisant une méthode métaheuristique, de préférence évolutionniste, de préférence un algorithme de recuit simulé. Un tel algorithme est bien connu pour l'optimisation non linéaire.

Si on a quitté le cyclage sur l'étape e2), sans qu'un score satisfaisant ait pu être obtenu, par exemple sans que le score ait pu atteindre ledit premier seuil, le procédé peut être arrêté (situation d'échec) ou une nouvelle étape 2) peut être lancée, avec une nouvelle information discriminante et/ou avec une nouvelle image actualisée. Le procédé peut être également poursuivi avec les conditions d'acquisition virtuelles correspondant au meilleur score atteint. Un avertissement peut être émis afin d'informer l'utilisateur de l'erreur sur le résultat.

Si on a quitté le cyclage sur l'étape e2) alors qu'un score satisfaisant a pu être obtenu, par exemple parce que le score a atteint, voire dépassé ledit premier seuil, les conditions d'acquisition virtuelles correspondent sensiblement aux conditions d'acquisition réelles.

De préférence, les conditions d'acquisition virtuelles comprennent les paramètres de calibration de l'appareil d'acquisition. Le procédé conduit permet ainsi d'évaluer les valeurs de ces paramètres sans qu'il soit nécessaire de connaître la nature de l'appareil d'acquisition ou son réglage. L'acquisition des images actualisées peut donc être réalisée sans précaution particulière, par exemple par le patient lui-même au moyen de son téléphone portable.

En outre, la recherche de la calibration réelle est effectuée en comparant une image actualisée avec des vues d'un modèle de référence à tester dans des conditions d'acquisition virtuelles que l'on teste. Avantageusement, elle ne nécessite pas que l'image actualisée fasse apparaître une jauge étalon de calibration, c'est-à-dire une jauge dont on connaît précisément les caractéristiques permettant de déterminer la calibration de l'appareil d'acquisition.

Comme cela apparaît clairement à présent, les images actualisées ne servent pas à créer un modèle tridimensionnel actualisé totalement nouveau, mais seulement à modifier le modèle de référence initial, très précis. Un modèle tridimensionnel actualisé totalement nouveau créé à partir de simples photographies prises sans précautions particulières serait en effet trop imprécis pour qu'une comparaison avec le modèle de référence initial puisse conduire à des conclusions sur le déplacement des dents.

Même si les conditions d'acquisition virtuelles correspondent exactement aux conditions d'acquisition réelles, des différences peuvent subsister entre les images actualisée et de référence si des dents se sont déplacées entre la création du modèle de référence initial et l'acquisition des images actualisées. La corrélation entre les images actualisée et de référence peut alors être encore améliorée en reprenant l'étape e2) après modification du modèle de référence à tester par déplacement d'un ou plusieurs modèles de dents (étape e3)). C'est l'objet de la deuxième optimisation.

La recherche du modèle de référence approximant au mieux le positionnement des dents lors de l'acquisition de l'image actualisée peut être effectuée comme la recherche des conditions d'acquisition virtuelles approximant au mieux les conditions d'acquisition réelles (étape e2)).

En particulier, le score est évalué au moyen d'une deuxième fonction d'évaluation. La deuxième fonction d'évaluation permet de décider si le cyclage sur les étapes e2) et e3) doit être poursuivi ou stoppé. La deuxième fonction d'évaluation peut par exemple être égale à 0 si le cyclage doit être stoppé ou être égale à 1 si le cyclage doit se poursuivre.

La valeur de la deuxième fonction d'évaluation dépend de préférence du meilleur score obtenu avec le modèle de référence à tester, c'est-à-dire des différences entre les cartes actualisée et de référence, dans les conditions d'acquisition virtuelles approximant au mieux lesdites conditions d'acquisition réelles.

La valeur de la deuxième fonction d'évaluation peut également dépendre du meilleur score obtenu avec un ou plusieurs modèles de référence testés précédemment.

Par exemple, il peut être décidé de poursuivre le cyclage si le score ne dépasse pas un deuxième seuil minimal. La valeur de la deuxième fonction d'évaluation peut également dépendre de paramètres aléatoires et/ou du nombre de cycles des étapes e2) et e3) déjà effectués.

Les fonctions d'évaluation classiquement utilisées dans les procédés d'optimisation métaheuristiques, de préférence évolutionnistes, en particulier dans les procédés de recuit simulé, peuvent être utilisées pour la deuxième fonction d'évaluation.

Si la valeur de la deuxième fonction d'évaluation indique qu'il est décidé de poursuivre le cyclage sur les étapes e2) et e3), on modifie le modèle de référence à tester et on recommence un cycle (étapes e2) et e3)) avec le nouveau modèle de référence à tester.

La modification du modèle de référence à tester correspond à un déplacement d'un ou plusieurs modèles de dents. Cette modification peut être aléatoire. La modification est de préférence guidée par des règles heuristiques, par exemple en favorisant les modifications qui, d'après une analyse des précédents scores obtenus, apparaissent les plus favorables pour augmenter le score.

De préférence, on recherche le déplacement d'un modèle de dent qui a le plus fort impact sur le score, on modifie le modèle de référence à tester en déplaçant ce modèle de dent, puis on poursuit le cyclage sur les étapes e2) et e3) de manière à optimiser le score. On peut ensuite rechercher, parmi les autres modèles de dent, celui qui a le plus fort impact sur l'amélioration du score, et à nouveau rechercher le déplacement optimal de cet autre modèle de dent sur le score. On peut poursuivre ainsi avec chaque modèle de dent.

Ensuite, il est possible de reprendre un cycle sur l'ensemble des modèles de dent et de poursuivre ainsi jusqu'à l'obtention d'un score supérieur au deuxième seuil. Bien entendu, d'autres stratégies peuvent être utilisées pour déplacer un ou plusieurs modèles de dent dans le modèle de référence à tester et rechercher le score maximal.

Le cyclage sur les étapes e2) et e3) est poursuivi jusqu'à ce que la valeur de la deuxième fonction d'évaluation indique qu'il est décidé de sortir de ce cyclage et de poursuivre à l'étape f), par exemple si le score atteint ou dépasse ledit deuxième seuil.

Le cyclage sur les étapes e2) et e3) permet avantageusement d'améliorer l'évaluation des paramètres de calibration de l'appareil d'acquisition à l'étape 1).

La recherche d'un modèle de référence avec un cyclage sur les étapes e2) et e3) pour rechercher les positions des modèles de dent qui optimisent le score est de préférence effectuée en utilisant une méthode métaheuristique, de préférence évolutionniste, de préférence un algorithme de recuit simulé. Un tel algorithme est bien connu pour l'optimisation non linéaire.

Si on a quitté le cyclage sur les étapes e2) et e3) sans qu'un score satisfaisant ait pu être obtenu, par exemple sans que le score ait pu atteindre ledit deuxième seuil, le procédé peut être arrêté (situation d'échec) ou repris en début d'étape 3) avec une nouvelle information discriminante et/ou avec une nouvelle image actualisée.

S'il est décidé de relancer le procédé en début d'étape 3) à partir d'une autre information discriminante et/ou d'une autre image actualisée parce que le premier seuil ou le deuxième seuil n'a pas été atteint, le choix de la nouvelle information discriminante et/ou de la nouvelle image actualisée peut dépendre des scores obtenus précédemment, afin de favoriser l'information discriminante et/ou l'image actualisée qui, au regard de ces scores, apparaissent les plus prometteuses.

Une nouvelle information discriminante, obtenue par exemple par combinaison d'autres informations discriminantes déjà testées, peut être utilisée. Le cas échéant, il peut être également demandé d'acquérir une ou plusieurs nouvelles images actualisées. De préférence, on fournit des indications permettant de guider le positionnement de l'appareil d'acquisition pour la capture de cette nouvelle image actualisée. Par exemple, on peut indiquer au patient qu'il devrait prendre une photo de la partie droite de son arcade inférieure.

Si on a quitté le cyclage sur les étapes e2) et e3) sans qu'un score satisfaisant ait pu être obtenu, un avertissement peut être émis afin d'informer l'utilisateur de l'erreur sur le résultat.

Si on a quitté le cyclage sur les étapes e2) et e3) alors qu'un score satisfaisant a pu être obtenu, par exemple parce que le score a atteint, voire dépassé ledit deuxième seuil, les conditions d'acquisition virtuelles correspondent sensiblement aux conditions d'acquisition réelles et les modèles de dents dans le modèle de référence obtenu (dit « modèle de référence actualisé ») sont sensiblement dans la position des dents du patient au moment de l'étape 2).

La fabrication du modèle de référence actualisé à l'étape 2) est avantageusement possible sans précaution particulière, notamment parce que le positionnement réel des dents est mesuré avec un modèle de référence actualisé qui résulte d'une déformation du modèle de référence initial afin que les images actualisées soient des vues du modèle de référence initial déformé.

A l'issue de l'étape 3), le modèle de référence actualisé concorde sensiblement aux images actualisées. Il est alors possible de faire des mesures précises sur le positionnement des dents et/ou sur leur forme.

Par ailleurs, l'étape 3) conduit, au moyen de première et deuxième cartes actualisées, et de première et deuxième cartes de référence, à la détermination des premières et deuxièmes conditions d'acquisition virtuelles permettant, en observant le modèle de référence actualisé, d'obtenir des première et deuxième images de référence présentant une concordance maximale avec les première et deuxième images actualisées, respectivement.

Cependant, le modèle de référence actualisé 20 (représenté très schématiquement sur la figure la) peut comporter des zones blanches 22, résultant par exemple d'une rétractation de la gencive ou d'un déplacement d'une dent ayant conduit à exposer une surface qu'il n'était pas possible de scanner lors de la création du modèle de référence initial. Visuellement, le modèle de référence actualisé présente ainsi des trous, par exemple lorsque le contour d'une dent est interrompu à proximité de la gencive.

**A l'étape 4**), on enrichit le modèle de référence actualisé au moyen d'informations contenues dans les première et deuxième images actualisées et qui n'étaient pas disponibles lors de la création du modèle de référence initial.

Dans un mode de réalisation, le dit procédé d'enrichissement comporte les étapes suivantes :
i) acquisition de première et deuxième images de ladite arcade, dites « images actualisées », dans des premières et deuxièmes conditions d'acquisition réelles faisant apparaître, sous des angles d'observation différents, une même région de ladite arcade ;
ii) comparaison de la première image actualisée avec une première image de référence du modèle de référence à enrichir, présentant une concordance maximale avec la première image actualisée, de manière à identifier au moins un point de la première image actualisée non représenté dans le modèle de référence à enrichir, ou « premier point orphelin » ;
iii) comparaison de la deuxième image actualisée avec une deuxième image de référence du modèle de référence à enrichir, présentant une concordance maximale avec la deuxième image actualisée, de manière à identifier un point de la deuxième image actualisée représentant un même point de l'arcade que le premier point orphelin, ou « deuxième point orphelin » ;
iv) ajout, dans le modèle de référence à enrichir, d'un point, dit « point parent », à un emplacement compatible avec les premier et deuxième points orphelins, de manière à obtenir un modèle de référence enrichi.

**L'étape i)** comprend une ou plusieurs caractéristiques de l'étape 2).

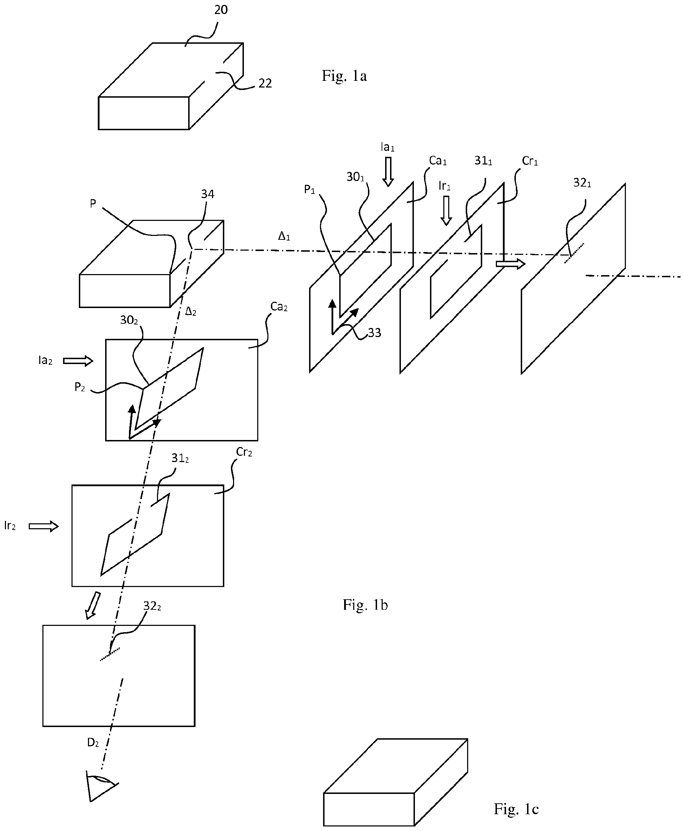

La figure 1 illustre une étape 4) qui comporte des étapes ii) à iv).

Les étapes ii) et iii) illustrent le mode de réalisation (a).

**A l'étape ii),** on compare la première image actualisée avec la première image de référence, de manière à identifier les premiers points orphelins, c'est-à-dire représentés sur la première image actualisée et non représentés dans le modèle de référence actualisé.

De préférence, cette comparaison utilise les premières cartes actualisée et de référence.

Dans le mode de réalisation de la figure 1, l'information discriminante est une information de contour.

La première carte actualisée Ca₁, obtenue à partir de la première image actualisée Ia₁, représente un premier contour « actualisé » 301 fermé. La première image de référence Ir₁ est obtenue par observation du modèle de référence actualisé 20 dans les premières conditions d'acquisition virtuelles simulant les conditions d'acquisition réelles ayant permis d'acquérir la première image actualisée Ia₁. Son traitement conduit à la première carte de référence Cr₁ qui fait apparaître un premier contour « de référence » 311 interrompu. L'interruption du premier contour de référence correspond en effet à la zone blanche 22.

Pour comparer la première image actualisée avec la première image de référence, on superpose les premières cartes actualisée Ca₁ et de référence Cr₁, ce qui fait apparaître un ensemble de premiers points orphelins 32₁, représentés en trait pointillé.

L'identification des premiers points orphelins 32₁ ne permet cependant pas de déterminer la position des points parents 34 respectifs dans le modèle de référence actualisé 20. En particulier, pour un premier point orphelin, il n'est pas possible d'identifier précisément la position du point parent le long de la première droite Δ₁ passant par le premier point orphelin (alors que la première image actualisée est dans sa position de registre avec la première image de référence dans l'espace du modèle de référence actualisé, comme représenté sur la figure 1) et parallèle à la première direction d'observation D₁ du modèle de référence actualisé lors de l'acquisition de la première image de référence.

**A l'étape iii),** on effectue les mêmes opérations qu'à l'étape ii), mais avec la deuxième image actualisée Ia₂.

La deuxième carte actualisée Ca2, obtenue à partir de la deuxième image actualisée Ia₂, représente un deuxième contour actualisé 302 fermé. La deuxième image de référence Ir2 est obtenue par observation du modèle de référence actualisé 20 dans les deuxièmes conditions d'acquisition virtuelles simulant les conditions d'acquisition réelles ayant permis d'acquérir la deuxième image actualisée Ia₂. Son traitement conduit à une deuxième carte de référence Cr2 qui fait apparaître un deuxième contour de référence 312 interrompu. L'interruption du deuxième contour de référence correspond à la zone blanche 22.

Pour comparer la deuxième image actualisée avec la deuxième image de référence, on superpose les deuxièmes cartes actualisée Ca2 et de référence Cr2, ce qui fait apparaître un ensemble de deuxièmes points orphelins 322, représentés en trait pointillé.

Les première et deuxième images actualisées ont été choisies pour représenter une même région de l'arcade (la région qui produit la zone blanche du modèle de référence actualisé). Leur analyse a conduit aux première et deuxième images de référence qui représentent la même zone blanche 22 et, au moins à proximité de la zone blanche, les mêmes points de l'arcade. Il est donc possible d'identifier, sur les première et deuxième images actualisées, un couple de premier et deuxième points remarquables, P₁ et P₂ respectivement, qui représentent un même point remarquable P de l'arcade, par exemple une extrémité de dent ou un point de contact entre deux dents, dans la région à proximité de la zone blanche. Trois tels couples permettent de définir un référentiel 33 commun aux première et deuxième images actualisées. Autrement dit, à proximité de la zone blanche, un même point de l'arcade est représenté sur les première et deuxième images actualisées par des premier et deuxième points qui ont sensiblement, voire exactement, les mêmes coordonnées dans ce référentiel.

En particulier, des points de contour peuvent être utilisés comme points remarquables pour définir le référentiel 33.

De manière équivalente, la détermination du référentiel 33 peut également être réalisée au moyen des cartes actualisées et de référence.

Il existe un biais résultant des perspectives différentes entre les première et deuxième images actualisées. L'effet de ce biais peut être cependant réduit en utilisant des points proches de la zone blanche pour établir le référentiel 33.

Au moyen de ce référentiel 33, on recherche, pour chaque premier point orphelin, un deuxième point orphelin présentant les mêmes coordonnées ou des coordonnées sensiblement identiques. On associe ainsi les premiers et deuxièmes points orphelins pour définir des couples qui chacun permettent de définir un point parent 34.

Plus précisément, le point parent est à l'intersection entre la première droite Δ₁ et la deuxième droite Δ₂ passant par le deuxième point orphelin (alors que la deuxième image actualisée est dans la deuxième position de registre avec la deuxième image de référence dans l'espace du modèle de référence actualisé) et parallèle à la deuxième direction d'observation D₂ du modèle de référence actualisé lors de l'acquisition de la deuxième image de référence.

Si pour un premier point orphelin, aucun deuxième point orphelin présentant les mêmes coordonnées ou des coordonnées sensiblement identiques n'est trouvé, on abandonne ce premier point orphelin et on reprend l'étape ii) pour déterminer un autre premier point orphelin.

Les étapes ii) et iii) sont de préférence réalisées pour tous les premiers points orphelins.

Le mode de réalisation (b) permet avantageusement de définir le point parent sans recourir à une deuxième image actualisée.

La position du point parent sur la première droite Δ₁ est déterminée en tenant compte de l'environnement de la zone blanche dont est issu le premier point orphelin.

De préférence, on construit, dans la zone blanche, une surface approximative s'étendant entre les bords de la zone blanche. La surface approximative peut être plane. De préférence, la surface approximative dépend de la forme de la surface du modèle de référence actualisé autour de la zone blanche. Par exemple, la surface approximative peut être déterminée pour rejoindre la surface du modèle de référence actualisé autour de la zone blanche sans former d'arête vive. De préférence encore, la surface approximative est déterminée de manière statistique ou par intelligence artificielle à partir de données historiques, acquises antérieurement à la mise en œuvre du procédé de l'invention.

Par exemple, si la zone blanche concerne une dent, par exemple une canine, il est possible d'établir la forme probable de cette dent en examinant les formes connues de dents « historiques » de même type (des canines). Suivant le mode de réalisation (b), on établit ainsi une surface approximative pour une région de la dent pour qu'elle soit en cohérence avec les données historiques.

Les données historiques peuvent en particulier comprendre des informations, de préférence des modèles de dent ou d'arcade, pour plus de 100, plus de 1000 ou plus de 10 000 patients.

L'intelligence artificielle utilise classiquement un dispositif d'apprentissage profond, en anglais « *deep learning* », sous la forme d'un réseau de neurones entrainé avec les données historiques, qui constituent une «base d'apprentissage ». Un réseau de neurones ou «réseau neuronal artificiel », est un ensemble d'algorithmes bien connu de l'homme de l'art.

Le réseau de neurones peut être en particulier choisi parmi :
- les réseaux spécialisés dans la classification d'images, appelés « CNN » (« Convolutional neural network »), par exemple
   - AlexNet (2012)
   - ZF Net (2013)
   - VGG Net (2014)
   - GoogleNet (2015)
   - Microsoft ResNet (2015)
   - Caffe: BAIR Reference CaffeNet, BAIR AlexNet
   - Torch:VGG_CNN_S,VGG_CNN_M,VGG_CNN_M_2048,VGG_CNN_M_10 24,VGG_CNN_M_128,VGG_CNN_F,VGG ILSVRC-2014 16-layer,VGG ILSVRC-2014 19-layer,Network-in-Network (Imagenet & CIFAR-10)
   - Google : Inception (V3, V4).
- les réseaux spécialisés dans la localisation, et détection d'objets dans une image, les Object Détection Network, par exemple:
   - R-CNN (2013)
   - SSD (Single Shot MultiBox Detector : Object Détection network), Faster R-CNN (Faster Region-based Convolutional Network method : Object Détection network)
   - Faster R-CNN (2015)
   - SSD (2015).

La liste ci-dessus n'est pas limitative.

L'entraînement d'un réseau de neurones consiste classiquement à activer les neurones qui le constituent. L'interconnexion de ces neurones définit ensuite l'architecture du réseau.

Par exemple, lorsque les données historiques sont des modèles d'arcade, on recherche les valeurs des paramètres du réseau qui, lorsqu'on soumet ces modèles d'arcade, dits « historiques », au réseau de neurones paramétré avec lesdites valeurs, lui permettent de déterminer des modèles d'arcade approximatifs qui se rapprochent le plus possible des modèles d'arcade historiques. Les modèles d'arcade approximatifs peuvent ainsi servir pour définir des surfaces approximatives ou des lignes, en particulier des lignes de contour, approximatives.

Alternativement ou en complément à la surface approximative, on peut construire, dans la zone blanche, une ligne approximative, par exemple rejoignant les extrémités d'un contour interrompu par la zone blanche. La forme de la ligne approximative est de préférence déterminée par analyse statistique ou par intelligence artificielle à partir de données historiques, comme expliqué ci-dessus.

De préférence, on détermine la position du point parent de manière à minimiser la distance entre le point parent et la première droite Δ₁.

**A l'étape iv),** les points parents sont ajoutés au modèle de référence actualisé.

L'emplacement, c'est-à-dire la position dans l'espace, d'un point parent peut être déterminé facilement. Un point parent est en effet à l'intersection des droites Δ₁ et Δ₂ passant par les premier et deuxième points orphelins, respectivement, et parallèles aux directions d'observation du modèle de référence actualisé pour obtenir les première et deuxième images actualisées, respectivement. Un tel emplacement est compatible avec les premier et deuxième points orphelins.

Si le modèle de référence était réellement ladite arcade, la représentation du point parent sur les première et deuxième images actualisées serait donc constituée par les premier et deuxième points orphelins.

De préférence, on ajoute un point parent 34 pour chaque couple de premier et deuxième points orphelins.

Le modèle de référence actualisé peut être ainsi complété.

De préférence, le modèle de référence initial est également enrichi en conséquence. A cet effet, il suffit de redéplacer les modèles de dents depuis leur position dans le modèle de référence actualisé jusqu'à leur position dans le modèle de référence initial, c'est-à-dire d'effectuer le déplacement inverse de celui effectué pour définir le modèle de référence actualisé.

L'enrichissement du modèle de référence initial n'est cependant possible que pour les contours de dents. Les tissus mous comme les gencives étant déformables, il n'est en effet pas possible de déterminer avec précision comment ils ont évolué entre l'instant initial et l'instant actualisé.

Le procédé est de préférence exécuté pour plusieurs couples de première et deuxième images actualisées, ce qui, avantageusement, améliore l'enrichissement.

Dans le mode de réalisation (b), le point parent peut être avantageusement retrouvé à partir de la seule première image.

Un dit procédé d'enrichissement peut encore comporter, après l'étape 4), une **étape 5)** consistant, pour chaque modèle de dent, à comparer les positionnements des modèles de dent dans le modèle de référence initial et dans le modèle de référence actualisé, afin de déterminer le déplacement des dents entre les étapes 1) et 2), et/ou de comparer les formes du modèle de référence initial et du modèle de référence actualisé, afin de déterminer la déformation et/ou le déplacement de dents entre les étapes 1) et 2).

Comme cela apparaît clairement à présent, l'invention fournit un système permettant d'enrichir un modèle de référence à enrichir, et en particulier un modèle de référence actualisé obtenu par déformation d'un modèle de référence initial. Avantageusement, cet enrichissement ne nécessite pas d'effectuer un nouveau scan des dents et n'oblige donc pas le patient à prendre un nouveau rendez-vous avec l'orthodontiste.

L'enrichissement du modèle de référence actualisé permet avantageusement de suivre le déplacement et/ou la déformation d'une région de l'arcade dentaire qui n'avait pas été scannée lors de la création du modèle de référence initial.

Bien entendu, l'invention n'est pas limitée au mode de réalisation décrit en détail et illustré. L'invention pourrait en particulier être mise en œuvre hors du cadre du procédé décrit dans WO 2016 066651.

Le système selon l'invention peut être utilisé pour enrichir un modèle de référence à enrichir, notamment pour qu'il soit plus complet et fournisse ainsi plus d'informations sur la situation dentaire du patient à l'instant où ce modèle de référence à enrichir a été créé.

En particulier, une zone blanche est une zone du modèle de référence à enrichir pour laquelle un point d'une image actualisée, en particulier un point du contour d'une dent, n'est pas représenté alors que, d'après une comparaison de l'image actualisée et du modèle de référence à enrichir, il devrait y figurer. A l'emplacement prévu pour ce point, le modèle de référence à enrichir présente donc un « trou ». Comme expliqué ci-dessus, le trou peut résulter du fait qu'il n'a pas été possible de scanner le point de la dent correspondant lors de la création du modèle de référence à enrichir. L'invention peut donc être mise en œuvre pour enrichir un modèle issu d'un scan incomplet, par exemple parce qu'une partie des dents était masquée lors du scan.

Le système selon l'invention peut être également utilisé pour adapter un modèle de référence à enrichir afin qu'il corresponde mieux à une situation orthodontique qui a évolué.

Le trou dans le modèle de référence à enrichir peut en particulier résulter du fait que la position et/ou la forme de la dent a évolué entre l'instant initial et l'instant actualisé, par exemple parce que la dent a subi une abrasion, par exemple lors d'un traitement orthodontique. L'invention peut alors être mise en œuvre pour évaluer une modification de la forme ou de la position d'une dent entre la création du modèle de référence à enrichir et l'étape i) d'acquisition des images actualisées. Les images actualisées peuvent être ainsi des images d'une dent dont la forme et/ou la position a évolué. Le procédé permet de construire, à partir d'un modèle de référence à enrichir représentant la dent dans sa position ou sa forme initiale, un modèle de référence enrichi représentant le contour de la dent à l'étape i). Dans un mode de réalisation préféré, le modèle de référence enrichi est comparé avec le modèle de référence à enrichir, ce qui permet avantageusement d'évaluer le déplacement et/ou la perte de matière résultant d'une abrasion.

Dans un mode de réalisation, les images actualisées sont acquises par exemple plus de 2, plus de 5 ou plus de 10 semaines avant la génération du modèle de référence à enrichir. Par exemple, le patient peut prendre des photos de ses dents puis, par exemple plusieurs mois après, un modèle de référence à enrichir peut être généré. L'enrichissement du modèle de référence à enrichir permet alors avantageusement de créer un modèle de référence à l'instant où les images actualisées ont été acquises. Avantageusement, le procédé permet ainsi de créer des modèles de situations dentaires passées.

Enfin, le patient n'est pas limité à un être humain. En particulier, un système selon l'invention peut être utilisé pour un autre animal.

## Revendications

1. Système comportant
- un appareil d'acquisition pour l'acquisition d'une première (Ia₁) et optionnellement une deuxième (Ia₂) image(s) d'une arcade dentaire d'un patient, dites première et deuxième image(s) actualisée(s), faisant apparaître une région de ladite arcade et acquise(s), dans des premières et deuxièmes conditions d'acquisition réelles respectivement, et
- un produit programme d'ordinateur comprenant des instructions de code de programme pour la mise en œuvre, lorsque le programme est exécuté par ordinateur, d'un procédé comportant les étapes suivantes :
B) exploration d'un modèle tridimensionnel numérique représentant ladite arcade dentaire du patient, dit modèle de référence à enrichir (20), de manière à déterminer une première vue du modèle de référence à enrichir, suivant une première direction d'observation (D1), ou première image de référence, ladite première image de référence présentant une concordance maximale avec la première image actualisée ;
C) détermination, par comparaison de la première image de référence et de la première image actualisée (Ia₁), d'un premier point orphelin (321) représenté sur la première image actualisée (Ia₁) et non représenté sur la première image de référence (Ir₁) lorsque la première image actualisée est dans une première position de registre dans laquelle elle est superposée, dans l'espace du modèle de référence à enrichir, avec la première image de référence (Ir₁) ;
D) ajout, dans le modèle de référence à enrichir (20), d'un point, dit point parent (34), sur une première droite (Δ₁) parallèle à la première direction d'observation et passant par le premier point orphelin (32₁) dans la première position de registre.

2. Système selon la revendication immédiatement précédente, comportant ladite deuxième image actualisée (Ia₂) et dans lequel, suivant ledit procédé, on détermine la position du point parent (34) sur la première droite (Δ₁) :
(a) comme étant la position la plus proche d'une deuxième droite (Δ₂) déterminée suivant les étapes suivantes :
- exploration du modèle de référence à enrichir (20) de manière à déterminer une deuxième vue du modèle de référence à enrichir, suivant une deuxième direction d'observation (D2), ou deuxième image de référence (Ir₂), ladite deuxième image de référence (Ir₂) présentant une concordance maximale avec la deuxième image actualisée (Ia₂) ;
- détermination d'un deuxième point orphelin (322) présentant les mêmes coordonnées que le premier point orphelin (321) dans un référentiel (33) commun aux première et deuxième images actualisées, la deuxième droite (Δ₂) étant la droite parallèle à la deuxième direction d'observation (D₂) et passant par le deuxième point orphelin (322) dans une deuxième position de registre dans laquelle la deuxième image actualisée (Ia₂) est superposée, dans l'espace du modèle de référence à enrichir, avec la deuxième image de référence (Ir₂) ;
ou
(b) en fonction d'une distance avec une surface approximative et/ou une ligne approximative définie, dans l'espace du modèle de référence à enrichir.

3. Système selon la revendication immédiatement précédente, dans lequel, suivant ledit procédé, le point parent est positionné de manière à minimiser ladite distance avec la surface approximative et/ou la ligne approximative.

4. Système selon l'une quelconque des deux revendications immédiatement précédentes, dans lequel, suivant ledit procédé, la surface approximative et/ou la ligne approximative sont déterminées par des méthodes statistiques ou au moyen d'un réseau de neurones.

5. Système selon l'une quelconque des revendications précédentes, dans lequel, suivant ledit procédé, on détermine la première et/ou deuxième image de référence au moyen d'une méthode métaheuristique.

6. Système selon l'une quelconque des revendications précédentes, dans lequel, suivant ledit procédé, pour déterminer au moins une desdites première et deuxième images de référence, on recherche des conditions d'acquisition virtuelles dans lesquelles l'observation du modèle de référence à enrichir fournit une image présentant une concordance maximale avec ladite au moins une desdites première et deuxième images actualisées, respectivement.

7. Système selon la revendication immédiatement précédente, dans lequel, suivant ledit procédé, on détermine ladite image de référence suivant les étapes suivantes :
a. traitement de l'image actualisée pour réaliser au moins une carte actualisée représentant, au moins partiellement, une information discriminante ;
b. détermination de conditions d'acquisition virtuelles à tester ;
c. réalisation d'une image de référence du modèle de référence dans lesdites conditions d'acquisition virtuelles à tester, ou image de référence à tester ;
d. traitement de l'image de référence à tester pour réaliser au moins une carte de référence représentant ladite information discriminante ;
e. comparaison des cartes actualisée et de référence de manière à déterminer une valeur pour une première fonction d'évaluation, ladite valeur pour la première fonction d'évaluation dépendant des différences entre lesdites cartes actualisée et de référence ;
f. en fonction de la valeur de la première fonction d'évaluation,
modification des conditions d'acquisition virtuelles à tester, puis reprise à l'étape c. ou
définition de ladite image de référence comme étant l'image de référence à tester.

8. Système selon la revendication immédiatement précédente, dans lequel, suivant ledit procédé, l'information discriminante est choisie dans le groupe constitué par une information de contour, une information de couleur, une information de densité, une information de distance, une information de brillance, une information de saturation, une information sur les reflets et des combinaisons de ces informations.

9. Système selon la revendication immédiatement précédente, dans lequel, suivant ledit procédé, l'information discriminante est une information de contour.

10. Système selon l'une quelconque des revendications précédentes, dans lequel, suivant ledit procédé, on recherche la première et/ou deuxième image de référence au moyen d'une méthode métaheuristique.

11. Système selon l'une quelconque des revendications précédentes, ledit procédé comportant, après l'étape D), une étape E) de comparaison du modèle de référence à enrichir avec le modèle de référence enrichi.

12. Système selon l'une quelconque des revendications précédentes, dans lequel, suivant ledit procédé, les étapes B) à D) sont exécutées, en boucle, pour plus de 5 couples de première et deuxième images actualisées.

## Patentansprüche

1. System, aufweisend
- eine Erfassungsvorrichtung für die Erfassung eines ersten (Ia₁) und wahlweise eines zweiten (Ia₂) Bilds eines Zahnbogens eines Patienten, die als erstes und zweites aktualisiertes Bild bezeichnet werden, die einen Bereich des Bogens erscheinen lassen und unter ersten beziehungsweise zweiten realen Erfassungsbedingungen erfasst werden, und
- ein Computerprogrammprodukt, das Programmcode-Anweisungen für die Umsetzung eines Verfahrens bei Ausführung des Programms durch einen Computer umfasst, das die folgenden Schritte aufweist:
B) Untersuchen eines digitalen dreidimensionalen Modells, das den Zahnbogen des Patienten darstellt, das als anzureicherndes Referenzmodell (20) bezeichnet wird, um eine erste Ansicht des anzureichernden Referenzmodells in einer ersten Betrachtungsrichtung (D1), oder erstes Referenzbild, zu bestimmen, wobei das erste Referenzbild eine maximale Konkordanz mit dem ersten aktualisierten Bild aufweist;
C) Bestimmen, durch Vergleich des ersten Referenzbilds und des ersten aktualisierten Bilds (Ia₁), eines ersten verwaisten Punkts (32₁), der auf dem ersten aktualisierten Bild (Ia₁) dargestellt und auf dem ersten Referenzbild (Ir₁) nicht dargestellt ist, wenn sich das erste aktualisierte Bild in einer ersten Registerposition befindet, in der es im Raum des anzureichernden Referenzmodells mit dem ersten Referenzbild (Ir₁) überlagert ist;
D) Hinzufügen, in dem anzureichernden Referenzmodell (20), eines übergeordneten Punkts (34) auf einer ersten Geraden (Δ₁), die parallel zur ersten Betrachtungsrichtung und in der ersten Registerposition durch den ersten verwaisten Punkt (32₁) verläuft.

2. System nach dem unmittelbar vorhergehenden Anspruch, aufweisend das zweite aktualisierte Bild (Ia₂), und wobei gemäß dem Verfahren die Position des übergeordneten Punkts (34) auf der ersten Geraden (Δ₁) wie folgt bestimmt wird:
(a) als die Position, die einer zweiten Geraden (Δ₂) am nächsten liegt, die gemäß den folgenden Schritten bestimmt wird:
- Untersuchen des anzureichernden Referenzmodells (20), um eine zweite Ansicht des anzureichernden Referenzmodells in einer zweiten Betrachtungsrichtung (D₂), oder zweites Referenzbild (Ir₂) zu bestimmen, wobei das zweite Referenzbild (Ir₂) eine maximale Konkordanz mit dem zweiten aktualisierten Bild (Ia₂) aufweist;
- Bestimmen eines zweiten verwaisten Punkts (32₂), der in einem gemeinsamen Bezugssystem (33) des ersten und zweiten aktualisierten Bilds dieselben Koordinaten wie der erste verwaiste Punkt (32₁) aufweist, wobei die zweite Gerade (Δ₂) die Gerade ist, die parallel zur zweiten Betrachtungsrichtung (D₂) verläuft und in einer zweiten Registerposition, in der das zweite aktualisierte Bild (Ia₂) im Raum des anzureichernden Referenzmodells mit dem zweiten Referenzbild (Ir₂) überlagert ist, durch den zweiten verwaisten Punkt (32₂) verläuft;
oder
(b) in Abhängigkeit von einem Abstand zu einer festgelegten ungefähren Fläche und/oder ungefähren Linie, im Raum des anzureichernden Referenzmodells.

3. System nach dem unmittelbar vorhergehenden Anspruch, wobei gemäß dem Verfahren der übergeordnete Punkt so positioniert ist, dass der Abstand zu der ungefähren Fläche und/oder ungefähren Linie minimiert wird.

4. System nach einem der zwei unmittelbar vorhergehenden Ansprüche, wobei gemäß dem Verfahren die ungefähre Fläche und/oder ungefähren Linie durch statistische Methoden oder mittels eines neuronalen Netzes bestimmt werden.

5. System nach einem der vorhergehenden Ansprüche, wobei gemäß dem Verfahren das erste und/oder zweite Referenzbild mittels einer metaheuristischen Methode bestimmt wird.

6. System nach einem der vorhergehenden Ansprüche, wobei gemäß dem Verfahren zum Bestimmen mindestens eines von dem ersten und zweiten Referenzbild virtuelle Erfassungsbedingungen gesucht werden, unter welchen die Betrachtung des anzureichernden Referenzmodells ein Bild liefert, das eine maximale Konkordanz mit dem mindestens einen von dem ersten bzw. zweiten aktualisierten Bild aufweist.

7. System nach dem unmittelbar vorhergehenden Anspruch, wobei gemäß dem Verfahren das Referenzbild gemäß den folgenden Schritten bestimmt wird:
a. Verarbeiten des aktualisierten Bilds, um mindestens eine aktualisierte Karte zu erstellen, die wenigstens teilweise eine unterscheidende Information darstellt;
b. Bestimmen von zu prüfenden virtuellen Erfassungsbedingungen;
c. Erstellen eines Referenzbilds des Referenzmodells unter den zu prüfenden Erfassungsbedingungen, oder zu prüfenden Referenzbilds;
d. Verarbeiten des zu prüfenden Referenzbilds, um mindestens eine Referenzkarte zu erstellen, die die unterscheidende Information darstellt;
e. Vergleichen der aktualisierten und der Referenzkarte, um einen Wert für eine erste Evaluierungsfunktion zu bestimmen, wobei der Wert für die erste Evaluierungsfunktion von den Differenzen zwischen der aktualisierten und der Referenzkarte abhängt;
f. je nach dem Wert der ersten Evaluierungsfunktion, Verändern der zu prüfenden virtuellen Erfassungsbedingungen, dann Wiederaufnahme bei Schritt c. oder
Festlegen des Referenzmodells als das zu prüfende Referenzbild.

8. System nach dem unmittelbar vorhergehenden Anspruch, wobei gemäß dem Verfahren die unterscheidende Information aus der Gruppe gewählt ist, die von einer Kontureninformation, einer Farbinformation, einer Dichteinformation, einer Abstandsinformation, einer Helligkeitsinformation, einer Sättigungsinformation, einer Information zu den Reflexen und Kombinationen dieser Informationen gebildet ist.

9. System nach dem unmittelbar vorhergehenden Anspruch, wobei gemäß dem Verfahren die unterscheidende Information eine Kontureninformation ist.

10. System nach einem der vorhergehenden Ansprüche, wobei gemäß dem Verfahren das erste und/oder zweite Referenzbild mittels einer metaheuristischen Methode gesucht wird.

11. System nach einem der vorhergehenden Ansprüche, wobei das Verfahren nach Schritt D) einen Schritt E) des Vergleichens des anzureichernden Referenzmodells mit dem angereicherten Referenzmodell aufweist.

12. System nach einem der vorhergehenden Ansprüche, wobei gemäß dem Verfahren die Schritte B) bis D) in einer Schleife für mehr als 5 Paare des ersten und zweiten aktualisierten Bilds ausgeführt werden.

## Claims

1. System comprising:
- an acquisition apparatus for the acquisition of a first image (Ia₁) and optionally of a second image (Ia₂) of a dental arch of a patient, said first image being called the first current image and said second image being called second current image, a region of said arch appearing in said image(s), said first image being acquired under first real acquisition conditions and said second image being acquired under second real acquisition conditions, and
- a computer program product comprising program-code instructions for implementing, when the program is executed by computer, a method comprising the following steps:
B) exploration of a three-dimensional digital model representing said dental arch of the patient, which is called the reference model to be enriched (20), in such a manner as to determine a first view of the reference model to be enriched, in a first direction of observation (D1), or first reference image, said first reference image exhibiting a maximum match with the first current image;
C) determination, by comparison of the first reference image and of the first current image (Ia₁), of a first orphan point (32₁) represented on the first current image (Ia₁) and not represented on the first reference image (Ir₁) when the first current image is in a first register position in which it is superposed, in the space of the reference model to be enriched, with the first reference image (Ir₁);
D) addition, in the reference model to be enriched (20), of a point, called the parent point (34), on a first straight line (Δ₁) parallel to the first direction of observation and passing through the first orphan point (32₁) in the first register position.

2. System according to the immediately preceding claim, comprising said second current image (Ia₂) and in which, in said method, the position of the parent point (34) on the first straight line (Δ₁) is determined:
(a) as being the closest position of a second straight line (Δ₂) determined according to the following steps:
- exploration of the reference model to be enriched (20) in such a manner as to determine a second view of the reference model to be enriched, in a second direction of observation (D₂), or second reference image (Ir₂), said second reference image (Ir₂) exhibiting a maximum match with the second current image (Ia₂);
- determination of a second orphan point (32₂) having the same coordinates as the first orphan point (32₁) in a reference frame (33) common to the first and second current images, the second straight line (Δ₂) being the straight line parallel to the second direction of observation (D₂) and passing through the second orphan point (32₂) in a second register position in which the second current image (Ia₂) is superposed, in the space of the reference model to be enriched, with the second reference image (Ir₂);
or
(b) as a function of a distance with an approximate surface and/or an approximate line defined in the space of the reference model to be enriched.

3. System according to the immediately preceding claim, in which, in said method, the parent point is positioned in such a manner as to minimize said distance with the approximate surface and/or the approximate line.

4. System according to either one of the two immediately preceding claims, in which, in said method, the approximate surface and/or the approximate line are determined by statistical methods or by means of a neural network.

5. System according to any one of the preceding claims, in which, in said method, the first and/or second reference image(s) is/are determined by means of a metaheuristic method.

6. System according to any one of the preceding claims, in which, in said method, in order to determine at least one of said first and second reference images, virtual acquisition conditions are sought under which the observation of the reference model to be enriched provides an image exhibiting a maximum match with said at least one of said first and second current images, respectively.

7. System according to the immediately preceding claim, in which, in said method, said reference image is determined according to the following steps:
a. processing of the current image in order to generate at least one current map representing, at least partially, a discriminating item of information;
b. determination of virtual acquisition conditions to be tested;
c. generation of a reference image for the reference model under said virtual acquisition conditions to be tested, or reference image to be tested;
d. processing of the reference image to be tested in order to generate at least one reference map representing said discriminating item of information;
e. comparison of the current and reference maps in such a manner as to determine a value for a first evaluation function, said value for the first evaluation function depending on the differences between said current and reference maps;
f. depending on the value of the first evaluation function,
modification of the virtual acquisition conditions to be tested, then return to the step c.
or
definition of said reference image as being the reference image to be tested.

8. System according to the immediately preceding claim, in which, in said method, the discriminating item of information is chosen from within the group consisting of contour information, color information, density information, distance information, brightness information, saturation information, information on reflections and of combinations of all these types of information.

9. System according to the immediately preceding claim, in which, in said method, the discriminating item of information is contour information.

10. System according to any one of the preceding claims, in which, in said method, the first and/or second reference image(s) is/are sought by means of a metaheuristic method.

11. System according to any one of the preceding claims, said method comprising, after step D), a step E) for comparison of the reference model to be enriched with the enriched reference model.

12. System according to any one of the preceding claims, in which, in said method, the steps B) to D) are executed, in a loop, for more than 5 pairs of first and second current images.
